# EUROPÄISCHE PATENTSCHRIFT

(11) **EP 1 562 860 B1**
(45) Veröffentlichungstag und Bekanntmachung des Hinweises auf die Patenterteilung: **27.04.2011**
(21) Anmeldenummer: 03779709.9
(22) Anmeldetag: 19.11.2003
(51) Int. Cl.: C01F 11/46, C01F 5/40, C09C 1/02

(54) **VERFAHREN ZUR HERSTELLUNG VON ERDALKALISULFATNANOPARTIKELN**
METHOD FOR PRODUCING ALKALINE EARTH SULPHATE NANOPARTICLES
PROCEDE POUR PRODUIRE DES NANOPARTICULES DE SULFATE ALCALINOTERREUX

(30) Priorität: 21.11.2002 DE 10254567
(43) Veröffentlichungstag der Anmeldung: 17.08.2005
(73) Patentinhaber: Centrum für Angewandte Nanotechnologie (CAN) GmbH, 20146 Hamburg (DE)
(72) Erfinder: IBARRA, Fernando, 20357 Hamburg (DE); MEYER, Christiane, 20357 Hamburg (DE); HAUBOLD, Stephan, 53229 Bonn (DE); HEIDELBERG, Thorsten, 22525 Hamburg (DE)
(74) Vertreter: HOFFMANN EITLE
(86) Internationale Anmeldenummer: PCT/DE2003/003823
(87) Internationale Veröffentlichungsnummer: WO 2004/046035

(56) Entgegenhaltungen:
- WO-A-01/07487
- DE-A- 10 005 685
- DE-A- 10 026 791
- DATABASE WPI Week 9418 Derwent Publications Ltd., London, GB; AN 94-147677 XP001165595 & JP 06 092630 A (KAOS CORP) 5. April 1994 (1994-04-05)
- DATABASE WPI Section Ch, Week 199306 Derwent Publications Ltd., London, GB; Class A85, AN 1993-048718 XP002296676 & JP 04 372712 A (MATSUSHITA ELEC IND CO LTD) 25. Dezember 1992 (1992-12-25)
- DATABASE CHEMABS [Online] CHEMICAL ABSTRACTS SERVICE, COLUMBUS, OHIO, US; SUMMERS, MARK ET AL: "Formation of BaSO4 Nanoparticles in Microemulsions with Polymerized Surfactant Shells" XP002296674 gefunden im STN Database accession no. 136:391444 & LANGMUIR , 18(12), 5023-5026 CODEN: LANGD5; ISSN: 0743-7463, 2002,
- DATABASE CHEMABS [Online] CHEMICAL ABSTRACTS SERVICE, COLUMBUS, OHIO, US; REES, GARETH D. ET AL: "Formation and Morphology of Calcium Sulfate Nanoparticles and Nanowires in Water-in-Oil Microemulsions" XP002296675 gefunden im STN Database accession no. 130:228259 & LANGMUIR , 15(6), 1993-2002 CODEN: LANGD5; ISSN: 0743-7463, 1999,

## Beschreibung

Die vorliegende Erfindung liegt auf dem Gebiet der Synthese von Nanopartikeln. Sie betrifft insbesondere Herstellungsverfahren für Nanopartikel mit einem im wesentlichen aus Z-sulfat (Z = Magnesium (Mg), Calcium (Ca), Strontium (Sr), Barium (Ba)oder binäre Mischungen derselben) bestehenden Gitter, bei dem Nanopartikel durch Kristallwachstum aus einer Z-Ionenquelle und einer Sulfationenquelle in einer Flüssigphasensynthesemischung synthetisiert werden.

Herkömmliche Herstellungsverfahren von Z-Sulfat (ZSO₄ ) Partikeln werden meist als Fällungsreaktion in wässriger Lösung durchgeführt. Die resultierende Größe der Primärpartikel reicht von etwa 100 Nanometern bis in den Millimeter-Bereich. Bedingt durch das Herstellungsverfahren sind diese Partikel agglomeriert und nicht homogen in jeder gewünschten Matrix dispergierbar.

ZSO₄ ist ein bekanntes Wirtsgitter für Dotanden und findet Verwendung in der Leuchtstoffindustrie. Außerdem ist es ein wichtiges Füllmaterial in der Kunststoffindustrie. Bisher hergestellte Partikelagglomerate sind so groß, dass sie das optische Verhalten, wie Transparenz des Kunststoffes deutlich verändern und nicht störungsfrei in die Polymermatrix eingebaut werden können. Mit homogen verteilten Nanopartikeln, deren Oberfläche auf die jeweilige Matrix angepasst wird, lassen sich diese Probleme umgehen. Eine weitere Verwendung findet insbesondere das BaSO₄ als Kontrastmittel für die X-Ray - Analyse.

DE 100 05 685 offenbart ein Herstellungsverfahren für Bariumsulfatnanopartikel mit mittlerem Teilchendurchmesser d50 von 100 bis 10.000 Nanometern. Die Teilchen werden in wässriger Lösung, bevorzugt in Natriumsulfat (Na2S04) - oder Schwefelsäure (H2SO4) -haltiger Lösung hergestellt.

Ein Nachteil bei dieser Methode besteht darin, dass keine Teilchen mit geringerem mittleren Teilchendurchmesser hergestellt werden können, ohne sie zumindest einem weiteren Verfahrensschritt, nämlich einem Mahlprozeß (Nassmahlprozeß) zu unterziehen.

Ein weiterer Nachteil liegt darin, dass die synthetisierten Teilchen direkt nach dem Ausfällen aus ihrer Synthesemischung nicht frei in Wasser dispergierbar sind. In DE 100 05 685 wird zum Erreichen der Dispergierbarkeit in Wasser vorgeschlagen, ein organisches Additiv auf die Oberfläche der Teilchen in einem weiteren Verfahrensschritt aufzubringen. Für diesen weiteren Verfahrensschritt werden drei Alternativen offenbart, wobei:
A) der abgetrennte Bariumsulfat-Filterkuchen erst zu einer Paste verarbeitet wird, die dann mit dem Additiv vermischt wird, oder
B) der Filterkuchen in Wasser suspendiert wird, und die Suspension mit dem Additiv vermischt wird, oder
C) der Filterkuchen getrocknet und dann mit dem Additiv vermischt wird, wobei dies in einem Sprühmahlverfahren bevorzugt geschehen kann, bei dem das Additiv, wenn es in fester Form als Ausgangsstoff vorliegt, erst noch in Lösung gebracht werden muß.

Dieses Herstellungsverfahren ist aufgrund des nachgeschalteten Verfahrensschrittes zum Aufbringen des Additivs umständlich und hat eine nicht befriedigende Ausbeute, zumindest bedingt durch das nachgeordnete Mahlverfahren.

Aufgabe der vorliegenden Erfindung ist es daher, zumindest Bariumsulfatnanopartikel mit wesentlich geringerem mittlerem Teilchendurchmesser auf einfache Weise zu erzeugen, wobei die Teilchen in Wasser homogen dispergierbar sein sollen.

DE 100 26 791 A betrifft ein Verfahren zur Herstellung von partikelförmigem Bariumsulfat, worin man eine Bariumsalzlösung mit einer Sulfatsalzlösung unter Bildung eines Reaktionsgemisches in einem kontinuierlich arbeitenden Mischreaktor vereinigt, in welchem Scher-, Schub- und Reibungskräfte von ineinandergreifenden Werkzeugen mit hoher Relativgeschwindigkeit auf die gebildete Reaktionsmischung einwirken. Die Synthese führt zu nanopartikulärem Bariumsulfat in einer Partikelgröße unterhalb von 100 nm, vorzugsweise unterhalb von 50 nm. Die Synthese findet in Wasser statt, wobei gemäß einer Ausführungsform eine kleine Menge eines Netz- oder Dispergiermittels während oder nach der Ausfällung zugesetzt werden kann. Unter den Dispergiermitteln werden neben Polyacrylaten, die in Beispiel 3 zu einer Teilchengröße von 30 nm führen, auch Polyether wie Polyglycolether und Amine, wie Triethanolamin aufgeführt.

Summers, M. et al.: "Formation of BaSO4 Nanoparticles in Microemulsions with Polymerized Surfactant Shells", Langmuir 18(12), 5023-5026 (2002) betrifft die Synthese von Bariumsulfat-Nanopartikeln in Wasser-in-Ölmikroemulsionen. Die Untersuchungen wurden mit polymerisierbaren kationischen Tensiden, offenbar Dodecyl-(11 Methacryloyloxy)dimethylammoniumbromid durchgeführt. Sowohl in Ethyl-nonanoat- als auch 50:50 Ethylnonanoat/Cyclohexan-Microemulsionströpfchen beobachtet man die Bildung von kugelförmigen Nanoteilchen mit hoher Monodispersität. Rees, G. et al.: "Formation and Morphology of Calcium Sulfate Nanoparticles and Nanowires in Water-in-Oil Miroemulsions", Langmuir, 15 (6), 1993-2002 (1999) betrifft ähnlich wie die Veröffentlichung von M. Summers die Herstellung von nanopartikulären Metallsulfaten, insbesondere Kalziumsulfat und Bariumsulfat in Wasser-in-Öl-Mikroemulsionen, die mit nichtionischen oder ionischen Tensiden stabilisiert wurden.

### VORTEILE DER ERFINDUNG

Der Gegenstand der unabhängigen Ansprüche löst diese Aufgabe, nicht nur für Bariumsulfatnanopartikel, sondern auch für andere Erdalkalisulfatnanopartikel, nämlich Magnesium-, Calcium- und Strontiumsulfatnanopartikel, oder für Nanopartikel aus binären Mischungen derselben.

Erfindungsgemäß wird ein Herstellungsverfahren für Nanopartikel offenbart, mit einem im wesentlichen aus Erdalkalisulfat, also "Z-sulfat" (Z = Magnesium (Mg), Calcium (Ca), Strontium (Sr), oder Barium (Ba)), - oder aus binären Mischungen derselben in einem beliebigen Mischungsverhältnis - bestehenden Gitter, bei dem Nanopartikel durch Kristallwachstum aus einer Z-Ionenquelle und einer Sulfationenquelle in einer Flüssigphasensynthesemischung synthetisiert werden, das dadurch gekennzeichnet ist, dass für die Synthese der Nanopartikel ein nicht-wässriges Lösemittel mit koordinierenden Eigenschaften verwendet wird, das als Steuerkomponente für das Partikelwachstum dient.

Die aus der Synthese resultierenden Partikel sind 0,5 bis 50 Nanometer (nm) groß, bevorzugt 2-30 nm und besonders bevorzugt 5-20 nm mit einer Größenverteilung von 50%, bevorzugt 20%, besonders bevorzugt 10-15%.

Je nach gewählter Reaktionstemperatur, Reaktionsdauer oder Reaktandenkonzentration erhält man rezeptur-spezifisch unterschiedlich große Partikel. Die Größenverteilung hängt speziell von dem verwendeten Molekül zur Wachstumssteuerung und dem verwendeten Lösemittel ab. Dabei besteht eine zentrale Funktion des koordinierenden Lösemittels darin, das Kristallwachstum im Vergleich zur Synthese ohne koordinierenden Lösemittel zu verlangsamen, so dass es grundsätzlich labortechnisch möglich wird, auch den Faktor Zeit als Verweildauer der Nanopartikel in der Synthesemischung als Steuerparameter neben den oben erwähnten rezeptur-spezifischen Parametern zur Steuerung des Größenwachstums mit einzubringen.

Kurz zusammengefasst werden bei der vorliegenden Erfindung koordinierende Lösemittel wie Glyzerin, Ethylenglycol und andere Polyethylenglycole, Polyalkohole oder Dimethylsulfoxid (DMSO) verwendet. Dazu wird das Barium bevorzugt als Chlorid und die Sulfatquelle bevorzugt als Tetrabutylammoniumhydrogensulfat vorgelegt. Weitere, optional in die Gitter der Nanopartikel während der Synthese einbaubare Metalldotanden werden ebenfalls bevorzugt als Chloride eingesetzt.

Die erfindungsgemäß gewonnenen Nanopartikel sind im Vergleich zum Stand der Technik extrem klein mit mittlerem Durchmesser zwischen 2 und 50 Nanometern herstellbar und lassen sich in besonders bevorzugter Weise ohne weitere Nachbehandlung homogen in Wasser dispergieren. Dies stellt einen erheblichen Vorteil gegenüber dem eingangs zitierten Verfahren des Standes der Technik dar, da dort erheblicher Aufwand getrieben werden muss, um die bei der Synthese zu großen (Mikrometer Durchmesser) agglomerierten Partikel durch Nassmahlen auf kleine Größe ( bis zu 100 Nanometer ) zu bringen. Darüber hinaus lassen sie sich gegebenenfalls auch nach einer Nachbehandlung auch gut in vielen anderen Lösemitteln dispergieren. Beispielhaft seien genant Toluol und Chloroform.

In den Unteransprüchen finden sich vorteilhafte Weiterbildungen und Verbesserungen des jeweiligen Gegenstandes der Erfindung.

Die Synthesemischung kann darüber hinaus auch ein nicht-koordinierendes Lösemittel enthalten. Mit dessen Anteil am Gesamtlösemittel kann die Teilchengröße einstellt werden. Im Grundsatz gilt: Je weniger von koordinierenden Lösemittel verwendet wird, desto größer werden die Partikel.

Wenn der überwiegende Teil des Lösemittels der Synthesemischung koordinierende Eigenschaften besitzt, können in vielen Fällen Nanopartikel unter 50 nm mittlerem Durchmesser hergestellt werden.

Vorteilhaft beziehungsweise zweckmäßig ist die Wahl der folgenden Ausgangsstoffe:
als Z-Quelle ZCl₂ * 2H₂O, ZBr₂ * 2H₂O, ZI₂ * 2H₂O oder Z (OH) ₂,
als Sulfatquelle Tetrabutylhammoniumhydrogensulfat, Bis(Trimethylsilyl)sulfat, Ammoniumhydrogensulfate des Typs R₁R₂R₃R₄NHSO₄, Ammoniumhydrogensulfat, Ammoniumsulfat, Alkalisulfate, Alkalihydrogensulfate, Amantadinsulfat, Ethylendiammoniumsulfat und Hydraziniumsulfat,
und als Dotandenquelle das jeweilige Metallnitrat, Metallbromid oder Metalliodid, bevorzugt das Metallchlorid.

Bei den bevorzugt verwendbaren Dotanden handelt es sich um eines der folgenden Ionen:
Eu(II), Sn(II),Mn(II), Sb(III), Pb(II), Zn(II), Ti(II), V(II), Cu(II), Cd(II), Ce(III), Sm(III), Pr(III), Nd(III), Gd(III), Tb(III), Dy(III), Ho(III), Er(III), Tm(III), Yb(III), Lu(III),Eu(III), Bi(III) Ag(I), Cu(I),
   oder einer beliebigen Kombination derselben, bevorzugt um die Kombination XY mit
X= Eu(II), Sn(II), Mn(II), Sb(III), Pb(II), Zn(II), Ti(II), V(II), Cu(II), Cd(II) und
Y= Ce(III), Sm(III), Pr(III), Nd(III), Gd(III), Tb(III), Dy(III), Ho(III), Er(III), Tm(III), Yb(III), Lu(III),Eu(III), Bi (III) Ag(I) und Cu(I).

Eine Nachbehandlung der fertigsynthetisierten Nanopartikel, die also ihre angestrebte Sollgröße erreicht haben, kann optional und in vorteilhafter Weise erfolgen, um die Oberfläche der Nanopartikel gezielt je nach späterem Anwendungszweck zu modifizieren, und damit dafür geeignet zu machen. Dabei lassen sich die Partikel durch eine anschließende chemische Modifikation der Oberfläche so bearbeiten, dass sie sich in jeder gewünschten Matrix homogen dispergieren lassen.

So können beispielsweise als Modifikationsmolekül für die Nanopartikeloberfläche
ein Phosphat, bevorzugt Trisethylhexylphosphat oder Tributylphosphat, ein Amin, bevorzugt Dodecylamin, ein Phosphonat, ein Phosphin, bevorzugt Trioctylphosphin, ein Phosphinoxid, bevorzugt Trioctylphosphinoxid, eine Carbonsäure, Alkohole, vorzugsweise mehrwertige Alkohole, organische Ester, Silane, Siloxane, organische Sulfone mit der Formel RSO₂R, organische Ketone (R-(C=O)-R), organische Nitrile (RCN), organische Sulfoxide (R₂-SO₂), organische Amide (R- (C=O) -NR' R oder R-(SO)-ONR'R) oder perfluorierte Modifikationen der oben genannten Substanzen, bevorzugt perfluorierte Alkohole und gegebenenfalls verwandte Stoffe
gezielt ausgesucht und als Modifizierungsmolekül verwendet werden, um die oberflächenmodifizierten Nanopartikel in einer entsprechenden Matrix, wie etwa Silikonöle, Teflone, Kunststoffe, Lacke, Farben, etc., die dann ähnlich zu der des Modifikationsmoleküls ausgewählt ist, homogen und fein dispergierbar zu machen.

Die hier vorgestellten Nanopartikel können dank der erfindungsgemäß erreichten geringen Größe nun intravenös appliziert werden, da sie homogen verteilt sind und ein Verstopfen von Venen, Arterien und anderen Blutgefäßen nicht zu erwarten ist. Dotiert mit paramagnetischen oder radioaktiven Elementen, können die Bariumsulfatnanopartikel als universeller Träger für die In-vivo-Diagnostik verwendet werden.

### KUZBESCHREIBUNG DER ZEICHNUNG

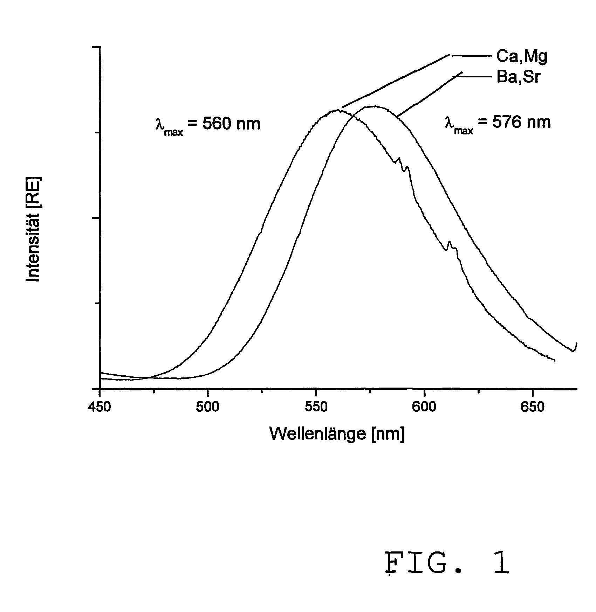

Fig. 1 zeigt die Manganemission in binären Erdalkalisulfat-Wirtsgittern (MSO4:Eu,Mn)

### BESCHREIBUNG DER AUSFÜHRUNGSBEISPIELE

Die verwendeten Ausgangsstoffe sind aus folgenden Bezugsquellen kommerziell erhältlich:
SIGMA ALDRICH Chemie GmbH, Deisenhofen, Deutschland, Firma MERCK, Darmstadt, Deutschland, und
Firma STREM, Karlsruhe, Deutschland.

### Ausführungsbeispiele:

### 1.

In einem Rundkolben werden 55g BaCl₂ eingewogen und in 300 ml Glycerin gelöst. Anschließend werden 30g in Glycerin gelöstem Imidazol zur Ba-haltigen Lösung gegeben und das Reaktionsgemisch anschließend unter leichtem Erwärmen 24 h getrocknet. Parallel werden 73g Tetrabutylammoniumhydrogensulfat in Glycerin gelöst und über Nacht getrocknet. Beide Lösungen werden dann bei 70 °C zusammengegeben und eine Stunde lang gerührt. Nach Zugabe von 0,5 Equivalenten Wasser bezogen auf Glyzerin, werden die somit synthetisierten Bariumsulfatteilchen mit Isopropanol gefällt mit Ethanol gewaschen und anschließend getrocknet. Es ergeben sich ca. 60 g Bariumsulfatnanopartikel mit homogener Grössenverteilung von 22 % um eine mittlere Teilchengrösse von 19 nm.

### 2.

Eine geringe Menge von 1g BaCl₂ und 0,15g MnCl₂ werden in Ethylenglykol gelöst und über Nacht bei 50 °C getrocknet. In einem zweiten Gefäß werden 0,07g EuCl₃, 0,54g Imidazol und 1,32g Tetrabutylammoniumhydrogensulfat gelöst und über Nacht bei Raumtemperatur (RT) getrocknet. Beide Lösungen werden anschließend bei RT vermischt und anschließend auf 180 °C zwei Stunden lang gerührt. Der entstandene Niederschlag wird anschließend abzentrifugiert und mit Methanol gewaschen. Es ergeben sich ca. 1 g BaSO4: Mn, Eu Nanopartikel, mit homogener Grössenverteilung von 15 % um eine mittlere Teilchengrösse von 10 nm. In vorteilhafter Weise wird EuCl₃ (EuCl sub3) als Ausgangsstoff verwendet, um EU(II) als Ionen in das Gitter einzubauen. Dies ist weitaus billiger und ermöglicht eine einfachere und erfolgreichere Synthese, als wenn EuCl₂ (EuCl sub2) als Ausgangsstoff verwendet würde. Das Verfahren lässt sich auch für größere Mengen gemäß dem geschilderten Verfahren durchführen.

### 3.

In einem Rundkolben werden 0,92g BaCl₂ und 0,54g Imidazol in einem 1:1 Gemisch aus Wasser und Methanol gelöst. Nach Zugabe von 25 ml Dimethylsulfoxid (DMSO) werden das Wasser und das Methanol bei RT und Vakuum abdestilliert. Anschließend werden nacheinander 0,044g Eu(II)Cl₂ und 1,32g Tetrabutylammoniumhydrogensulfat in DMSO dazugegeben. Anschließend wird die Reaktionsmischung 0,5 Stunden bei 170 °C gerührt.

Es ergeben sich ca. 1 g BaS04: Eu++ Nanopartikel, mit homogener Grössenverteilung von 20% um eine mittlere Teilchengrösse von 10 nm.

Das Verfahren lässt sich auch für größere Mengen gemäß dem geschilderten Verfahren durchführen.

### 4. Nachbehandlung :

Bariumsulfat hergestellt wie oben unter 1. beschrieben, wird in ausreichender Menge mit Dodecylamin als Modifizierungsmolekül - mindestens im Verhältnis 1:1 Gew.-prozent Bariumsulfat zu Modifizierungsmolekül zusammengebracht und anschließend bevorzugt unter Ausschluss von Sauerstoff auf Siedetemperatur des Dodecylamins, bevorzugt 100-300°C aufgeheizt und dort unter stetigem Rühren gehalten während 0,1-2 Stunden. Es ergibt sich Löslichkeit der Nanopartikel in Toluol.

### 5. binäre Mischung Magnesium/ Calcium: Mg, CaS04:Eu(II), Mn(II)

0,305g MgCl2, 0,329g CaCl2 und 0,158 g MnCl2 werden in 25 ml Ethylenglycol bei Raumtemperatur (RT) bis zur vollständigen Lösung der Metallsalze gerührt und anschli9eßend bei 50°C bei Vakuum über Nacht getrocknet.

Gleichzeitig werden 0,0446g EuCl2 mit 3ml Ethylenglycol 0,5446g in 3 ml Ethylenglycol und 1,3242g tetrabutylammoniumhydrogensulfat in 10 ml Ethylenglycol gelöst und über Nacht bei RT getrocknet. Anschließend werden Imidazollösung, Eu(II)-lösung und Sulfatlösung zu den Metallsalzen unter Stickstoff zugegeben. Anschließend wird die fertige Reaktionsmischung auf 180 °C geheizt und zwei Stunden gerührt. Der entstandene Niederschlag wird abzentrifugiert mit Methanol gewaschen und anschließend getrocknet. Die Partikelgröße beträgt 10 -15 nm und hat eine Größenverteilung von 10 - 20 %.

Das Verfahren lässt sich auch für größere Mengen gemäß dem geschilderten Verfahren durchführen. Erfindungsgemäß kann von einem weiteren Effekt profitiert werden: Die Zugabe von Magnesium (Mg) bewirkt eine Verschiebung der Manganemission und damit eine Veränderung des Farbeindruckes.

Durch das gitterbildende Erdalkaliion wird die Lage der Manganfluoreszenzbande bestimmt, so dass durch binäre Mischungen Verschiebungen der Bande hervorgerufen werden. Dies geht mit einer Veränderung des Farbeindrucks einher. Dieser Effekt ist beispielhaft in **Fig. 1** gezeigt, wobei sich eine Verschiebung der Emissionsbande um circa 16 nm ergibt, wobei sich das Emissionsmaximum von circa 576 nm auf 560 nm verschiebt.

Weitere Ausführungsbeispiele für die Herstellung von Calciumsulfatnanopartikeln ergeben sich aus den obigen Ausführungsbeispielen mittels Ersetzen des Bariums durch Calcium im stöchiometrisch angepasstem Verhältnis.

Weitere Ausführungsbeispiele für die Herstellung von Strontiumsulfatnanopartikeln ergeben sich aus den obigen Ausführungsbeispielen mittels Ersetzen des Bariums durch Strontium im stöchiometrisch angepasstem Verhältnis.

Weitere Ausführungsbeispiele für die Herstellung von Magnesiumsulfatnanopartikeln ergeben sich aus den obigen Ausführungsbeispielen mittels Ersetzen des Bariums durch Magnesium im stöchiometrisch angepasstem Verhältnis.

### Ende der Ausführungsbeispiele

### Verwendungen der hergestellten Nanopartikel:

Es sollte noch angemerkt werden, dass dank der vorliegenden Erfindung eine gezielte Auswahl der Dotanden nach ihren physikalischen Eigenschaften, wie etwa Paramagnetismus, Lichtabsorption oder -emission eine Verwendung des erfindungsgemäßen Z-sulfats mit einer mittleren Teilchengröße von 0,5-50 nm als intravenös oder intramuskulär applizierbares In-Vivo-Diagnostikum für die Röntgenanalyse, Computertomographieanalyse, Kernspintomographieanalyse oder für die Fluoreszenzanalyse in organischen Systemen, besonders für Pflanzen ermöglicht. Eine Inkorporierung vieler der prinzipiell in Reinsubstanz giftigen Dotanden sind - eingebaut im Wirtsgitter der Nanopartikel für den menschlichen Körper unschädlich. Durch die extrem geringe Teilchengröße können Markerflüssigkeiten, die diese Nanopartikel tragen, auch durch engste Querschnitte fliessen, ohne dass diese zur Verstopfung neigen.

Auch ist die Verwendung der erfindungsgemäßen Z-sulfatnanopartikel mit einer mittleren Teilchengröße von 0,5-50 nm als Träger und Wirtsgitter für radioaktive Isotope in der Isotopendiagnostik möglich.

Der erfindungsgemäß hergestellte Stoff Z-sulfat in Form der Nanopartikel mit einer mittleren Teilchengröße von 0,5-50 nm kann in vorteilhafter Weise auch als Füllstoff für kleinste Kunststoffteile, dünnste Folien, mit dem Ziel eines verbesserten mechanisches Verhaltens des Kunststoffes ohne Verlust der optischen Eigenschaften, des weiteren für Farben und Lacke, verwendet werden, ohne deren Fließfähigkeit oder andere Eigenschaften zu beeinflussen. Sie können auch für Produkte wie Autoreifen, Filmträger oder als Zwischenprodukt für Masterbatches und Compounds verwendet werden.

Obwohl die vorliegende Erfindung anhand eines bevorzugten Ausführungsbeispiels vorstehend beschrieben wurde, ist sie darauf nicht beschränkt, sondern auf vielfältige Weise modifizierbar.

Schließlich ist die vorliegende Erfindung nicht auf die vereinfachte Herstellung von Z-sulfatnanopartikeln bis 50 Nanometer beschränkt. Es lassen sich auch bei der Synthese von Z-sulfatnanopatrikeln mit Teilchendurchmesser d50 > 50 oder d50 > 100 Nanometer erhebliche Vorteile erzielen, da die Nanopartikel im Gegensatz zum Stand der Technik in einem 1-Schritt Verfahren hergestellt werden, was die Herstellung verbilligt und vereinfacht. Dafür werden die Nanopartikel länger in der Synthesemischung belassen.

Schließlich können die Merkmale der Unteransprüche im wesentlichen frei miteinander und nicht durch die in den Ansprüchen vorliegende Reihenfolge miteinander kombiniert werden, sofern sie unabhängig voneinander sind.

## Patentansprüche

1. Nanopartikel mit einem Kristallgitter oder im Falle einer Dotierung einem Wirtsgitter bestehend im Wesentlichen aus Z-sulfat worin Z = Magnesium (Mg), Calcium (Ca), Strontium (Sr), oder Barium (Ba), erhältlich durch Synthese in einem nicht-wässrigen Lösemittel mit koordinierenden Eigenschaften unter gesteuertem Kristallwachstum, wobei die Nanopartikel eine mittlere Teilchengröße von 0,2 bis 50 Nanometern besitzen und die Eigenschaft aufweisen, dass sie in Wasser dispergierbar sind.

2. Herstellungsverfahren für Nanopartikel mit einem Kristallgitter oder im Falle einer Dotierung einem Wirtsgitter bestehend im wesentlichen aus Z-sulfat worin Z = Calcium (Ca), Strontium (Sr), oder Barium (Ba), bei dem die Nanopartikel durch Kristallwachstum aus einer Z-Ionenquelle und einer Sulfationenquelle in einer Flüssigphasensynthesemischung synthetisiert werden, **dadurch gekennzeichnet,**
**dass** für die Synthese der Nanopartikel ein nicht-wässriges Lösemittel mit koordinierenden Eigenschaften verwendet wird, das als Steuerkomponente für das Wachstum der Partikel dient.

3. Herstellungsverfahren nach dem vorstehenden Anspruch 2, **dadurch gekennzeichnet, dass** das koordinierende Lösemittel unter Polyalkoholen und Dimethylsulfoxid (DMSO) ausgewählt wird.

4. Herstellungsverfahren nach dem vorstehenden Anspruch 3, worin der Polyalkohol unter Glycerin, Ethylenglycol und Polyethylenglycolen ausgewählt wird.

5. Herstellungsverfahren nach dem Anspruch 3 oder 4, wobei Kombinationen der koordinierenden Lösemittel verwendet werden.

6. Herstellungsverfahren nach Anspruch 2, 3, 4 oder 5, bei dem zur Synthese von dotierten Z-Sulfat-Nanopartikeln eine basisch wirkende Komponente, bevorzugt ein Amin, besonders bevorzugt Trioctylamin, oder noch bevorzugter Imidazol in die Synthesemischung gegeben wird.

7. Herstellungsverfahren nach dem vorstehenden Anspruch, wobei Dimethylsulfoxid (DMSO) als Lösemittel zur Synthese von Mangan- und Europium(II)-dotierten Z-Sulfatnanopartikeln verwendet wird, und Imidazol als Base beigegeben wird.

8. Herstellungsverfahren nach Anspruch 2, wobei die Synthesemischung darüber hinaus ein nicht-koordinierendes Lösemittel enthält.

9. Herstellungsverfahren nach dem vorstehenden Anspruch, worin in der Mischung aus dem nicht-wässrigen Lösemittel mit koordinierende Eigenschaften und dem nicht-koordinierenden Lösemittel das Lösemittel mit koordinierenden Eigenschaften überwiegt.

10. Verfahren nach Anspruch 2, wobei als Sulfationenquelle eines oder mehrere von:
Tetrabutylammoniumhydrogensulfat, Bis(Trimethylsilyl)sulfat, Ammoniumhydrogensulfate des Typs R₁R₂R₃R₄NHSO₄, Ammoniumhydrogensulfat, Ammoniumsulfat, Alkalisulfate, Alkalihydrogensulfate, Amantadinsulfat, Ethylendiammoniumsulfat und Hydraziniumsulfat verwendet wird.

11. Herstellungsverfahren nach Anspruch 2, weiter enthaltend den Nachbehandlungsschritt:
Erhitzen der synthetisierten Nanopartikel in Gegenwart eines Modifikationsmoleküls für die Oberfläche der Nanopartikel,
wobei das Modifikationsmolekül aus folgender Gruppe ausgewählt ist:
einem Phosphat bevorzugt Trisethylhexylphosphat oder
Tributylphosphat,
einem Amin bevorzugt Dodecylamin,
einem Phosphonat, einem Phosphin bevorzugt Trioctylphosphin,
einem Phosphinoxid bevorzugt Trioctylphosphinoxid,
einer Carbonsäure,
Alkohole, vorzugsweise mehrwertige Alkohole, Glycerin, Ethylenglycol, Polyglycole, organische Ester, Silane, Siloxane, organische Sulfone mit der Formel RSO₂R, organische Ketone (R-(C=O)-R), organische Nitrile (RCN), organische Sulfoxide (R₂-SO₂), organische Amide (R-(C=O)-NR'R oder R-(SO)-ONR'R) oder perfluorierte Modifikationen der vorgenannten Substanzen, bevorzugt perfluorierte Alkohole.

## Claims

1. Nanoparticles having a crystal lattice or, in the case of doping, a host lattice consisting essentially of Z sulphate, wherein Z = magnesium (Mg), calcium (Ca), strontium (Sr), or barium (Ba), which can be obtained by synthesis in a non-aqueous solvent with coordinating properties under controlled crystal growth, wherein the nanoparticles have an average particle size of 0.2 to 50 nanometres and have the property that they can be dispersed in water.

2. Production process for nanoparticles having a crystal lattice or, in the case of doping, a host lattice consisting essentially of Z sulphate, wherein Z = calcium (Ca), strontium (Sr), or barium (Ba), in which the nanoparticles are synthesised by crystal growth from a Z ion source and a sulphate ion source in a liquid phase synthesis mixture, **characterised in that** for the synthesis of the nanoparticles, a non-aqueous solvent having coordinating properties is used, which serves as control component for the growth of the particles.

3. Production process according to the preceding claim 2, **characterised in that** the coordinating solvent is selected from polyalcohols and dimethyl sulphoxide (DMSO).

4. Production process according to the preceding claim 3, wherein the polyalcohol is selected from glycerol, ethylene glycol and polyethylene glycols.

5. Production process according to claim 3 or 4, wherein combinations of the coordinating solvents are used.

6. Production process according to claim 2, 3, 4 or 5, in which to synthesise doped Z sulphate nanoparticles, a base-acting component, preferably an amine, particularly preferably trioctylamine, or more preferably imidazole, is added to the synthesis mixture.

7. Production process according to the preceding claim, wherein dimethyl sulphoxide (DMSO) is used as solvent for the synthesis of manganese-doped and europium(II)-doped Z sulphate nanoparticles, and imidazole is added as the base.

8. Production process according to claim 2, wherein the synthesis mixture furthermore contains a non-coordinating solvent.

9. Production process according to the preceding claim, wherein in the mixture of the non-aqueous solvent having coordinating properties and the non-coordinating solvent, the solvent having coordinating properties predominates.

10. Process according to claim 2, wherein as sulphate ion source, one or more of:
tetrabutylammonium hydrogen sulphate, bis(trimethylsilyl) sulphate, ammonium hydrogen sulphates of the type R₁R₂R₃R₄NHSO₄, ammonium hydrogen sulphate, ammonium sulphate, alkali metal sulphates, alkali metal hydrogen sulphates, amantadine sulphate, ethylene diammonium sulphate and hydrazinium sulphate is used.

11. Production process according to claim 2, further containing the post-treatment step:
heating the synthesised nanoparticles in the presence of a modification molecule for the surface of the nanoparticles, wherein the modification molecule is selected from the following group:
a phosphate, preferably tris-ethylhexyl phosphate or tributyl phosphate,
an amine, preferably dodecylamine,
a phosphonate, a phosphine, preferably trioctyl phosphine, a phosphine oxide,
preferably trioctyl phosphine oxide,
a carboxylic acid,
alcohols, preferably polyhydric alcohols, glycerol, ethylene glycol, polyglycols,
organic esters, silanes, siloxanes, organic sulphones having the formula RSO₂R, organic ketones (R-(C=O)-R), organic nitriles (RCN), organic sulphoxides (R₂-SO₂), organic amides (R-(C=O)-NR' R or R-(SO)-ONR'R) or perfluorinated modifications of the afore-mentioned substances, preferably perfluorinated alcohols.

## Revendications

1. Nanoparticules, avec un réseau cristallin ou, dans le cas d'un dopage, avec un réseau hôte composé essentiellement de sulfate de Z dans lequel Z = magnésium (Mg), calcium (Ca), strontium (Sr), ou barium (Ba), obtenues par synthèse dans un solvant non aqueux avec des propriétés de coordination sous une croissance cristalline commandée, les nanoparticules présentant une taille de particule moyenne dans la fourchette comprise entre 0,2 et 50 nanomètres et présentant la propriété d'être dispersibles dans l'eau.

2. Procédé de fabrication pour des nanoparticules avec un réseau cristallin ou, dans le cas d'un dopage, avec un réseau hôte composé essentiellement de sulfate de Z dans lequel Z = calcium (Ca), strontium (Sr), ou barium (Ba), dans lequel les nanoparticules sont synthétisées par croissance cristalline à partir d'une source d'ions Z et d'une source d'ions sulfate dans un mélange de synthèse en phase liquide, **caractérisé en ce qu'**
un solvant non aqueux ayant des propriétés de coordination, servant de composant de commande pour la croissance des particules, est utilisé pour la synthèse des nanoparticules.

3. Procédé de fabrication selon la revendication 2 précédente, **caractérisé en ce que** le solvant de coordination est sélectionné parmi les polyalcools et le diméthylsulfoxyde (DMSO).

4. Procédé de fabrication selon la revendication 3 précédente, **caractérisé en ce que** le polyalcool est sélectionné parmi la glycérine, l'éthylène glycol et les polyéthylène glycols.

5. Procédé de fabrication selon la revendication 3 ou 4, **caractérisé en ce que** sont utilisées des combinaisons des solvants de coordination.

6. Procédé de fabrication selon la revendication 2, 3, 4 ou 5, dans lequel, pour la synthèse de nanoparticules de sulfate de Z dopées, un composant à effet basique, de préférence une amine, de manière particulièrement préférée une trioctylamine, ou de manière encore mieux préférée un imidazol est fourni dans le mélange de synthèse.

7. Procédé de fabrication selon la revendication précédente, **caractérisé en ce que** le diméthylsulfoxyde (DMSO) est utilisé en tant que solvant pour la synthèse de de nanoparticules de sulfate de Z dopées au manganèse et à l'europium(II), et l'imidazol est fourni entant que base.

8. Procédé de fabrication selon la revendication 2, **caractérisé en ce que** le mélange de synthèse contient en plus un solvant non coordonnant.

9. Procédé de fabrication selon la revendication précédente, dans lequel, dans le mélange composé du solvant non aqueux ayant des propriétés de coordination et du solvant non coordonnant, le solvant ayant des propriétés de coordination est prédominant.

10. Procédé de fabrication selon la revendication 2, dans lequel est utilisé comme source d'ions sulfate un ou plusieurs parmi le sulfate de tétrabutylammoniumhydrogène, bis(triméthylsilyl) sulfate, sulfate d'hydrogène d'ammonium du type R₁R₂R₃R₄NHSO₄,
sulfate d'hydrogène d'ammonium, sulfate d' ammonium, sulfate d'alcali, sulfate d'alcali hydrogéné, sulfate d'amantadine, sulfate d'éthylène diammonium et sulfate d'hydrazinium.

11. Procédé de fabrication selon la revendication 2, comprenant en outre l'étape de retraitement suivante :
chauffage des nanoparticules synthétisées en présence d'une molécule de modification pour la surface des nanoparticules, la molécule de modification étant sélectionnée dans le groupe suivant :
un phosphate, de référence un triséthylhexylphosphate ou tributylphosphate,
une amine, de préférence une dodécylamine,
un phosphonate, une phosphine, de préférence une trioctylphosphine,
un oxyde de phosphine, de préférence un oxyde de trioctylphosphine,
un acide carboxylique,
des alcools, de préférence des alcools polyvalents, la glycérine, l'éthylène glycol, des polyglycols,
des esters organiques, silanes, siloxanes, sulfones organiques de formule RSO₂R, des cétones organiques (R-(C=O)-R), des nitriles organiques (RCN), des sulfoxydes organiques (R₂-SO₂), des amides organiques (R-(C=O)-NR'R ou R-(SO)-ONR'R) ou des modifications perfluorées des substances précitées, de préférence des alcools perfluorés.
